# EUROPEAN PATENT APPLICATION

(11) **EP 3 866 107 A1**
(43) Date of publication of application: **18.08.2021**
(21) Application number: 20157508.1
(22) Date of filing: 14.02.2020
(51) Int. Cl.: G06T 7/12, G06T 7/149

(54) **MODEL-BASED IMAGE SEGMENTATION**

(71) Applicant: Koninklijke Philips N.V., 5656 AG Eindhoven (NL)
(72) Inventor: BUERGER, Christian, 5656 AE Eindhoven (NL); KLINDER, Tobias, 5656 AE Eindhoven (NL); VON BERG, Jens, 5656 AE Eindhoven (NL); FRANZ, Astrid Ruth, 5656 AE Eindhoven (NL); LENGA, Matthias, 5656 AE Eindhoven (NL); LORENZ, Cristian, 5656 AE Eindhoven (NL)
(74) Representative: Philips Intellectual Property & Standards

(57) **Abstract**

Presented are concepts for initialising a model for model-based segmentation of an image which use specific landmarks (e.g. detected using other techniques) to initialize the segmentation mesh. Using such an approach, embodiments need not be limited to predefined model transformations, but can initialise a segmentation mesh with arbitrary shape. In this way, embodiments may provide for an image segmentation algorithm that not only delivers a robust surface-based segmentation result but also does so for strongly varying target structure variations (in terms of shape).

## Description

### FIELD OF THE INVENTION

The invention relates to the field of model-based image segmentation, and more particularly to initialising a model of a model space for model-based segmentation of an image.

### BACKGROUND OF THE INVENTION

Model-based image segmentation is used in a range of applications to automatically segment an object from an image. For example, model-based image segmentation techniques are used in medical image processing to segment an organ, or some other body part, from a volumetric medical image.

Model-based segmentation (MBS) techniques using triangulated surface meshes have proven to be fast (because the image data is only processed close to the mesh surface within a certain capture range), robust and accurate (due to encoded shape priors). In these techniques, a shape prior is encoded in a surface mesh, and the mesh is adapted to an image. The shape prior means that an object in an image can be segmented even if some parts of the object's boundary cannot be detected, and, since only image data close to the mesh surface is processed to adapt the mesh to the image, the image can be segmented quickly.

To enable the model to adapt to the image, it is generally preferable to initialise the model (of the model space) so that its adaptation to the image is within the capture range of the target structure (e.g. an organ in a medical image).

Common model initialisation techniques can be separated into the following two categories:
(I) Initialisation is configured such that it only allows a limited set of transformations that can be applied to the model, such as global scaling, translation, or a predefined set of other transformations. However, this might lead to 'out of capture range' problems when the target organ shape deviates strongly from what those transformations can capture with respect to the model's mean mesh.
(II) The initialisation uses non-rigid deformations of the model to a cloud of detected landmarks, e.g. using thin-place splines. However, this requires and limits the landmark detection to find landmarks only close to the target structure boundary (thus meaning other well-detectable landmarks far away from the target structure cannot be used).

There is therefore a need for a model initialisation technique that is not restricted to a limited set of transformations and not to landmarks close to the organ boundary, so that it can initialise a segmentation mesh with arbitrary shape for example.

### SUMMARY OF THE INVENTION

The invention is defined by the claims.

According to examples in accordance with an aspect of the invention, there is provided a computer-implemented method of initialising a model of a model space for model-based segmentation of an image.

The method comprises: defining a set of landmarks in a model space; for each of the landmarks, determining a respective connection to the model of the model space, the connection being representative of the position of the landmark relative to the model; detecting the set of landmarks in the image; and placing the model within the detected set of landmarks based on the determined connections for the landmarks.

Proposed are concepts for initialising a model for model-based segmentation of an image which use specific landmarks (e.g. detected using other techniques) to initialize the segmentation mesh. Using such an approach, embodiments need not be limited to predefined model transformations, but can initialise a segmentation mesh with arbitrary shape. In this way, embodiments may provide for an image segmentation algorithm that not only delivers a robust surface-based segmentation result but also does so for strongly varying target structure variations (in terms of shape).

For instance, it is proposed to use a set of landmarks that can be arbitrarily distributed over the image to initialize the MBS model by an arbitrary shape. By way of example, a set of landmarks can be defined and their spatial positions then correlated with respect to the MBS model. A landmark detector (using any known landmark detection algorithm) may then be used to find the set of landmarks in the image to segment. Once the landmarks are detected, the model can be 'activated' by placing the model shape into the landmark set according to the required positions of the landmarks relative to the model. During such placement, the model can be deformed (while considering the model's internal energy to only allow realistic deformations) in such a way that relative positions from model to landmarks are maintained. With the landmarks are being distributed around the target structure outline (e.g. with arbitrary distance), the initialization will be within a capture range of the model, and a successful segmentation can thus be ensured.

The proposed concept(s) may improve the initialization of MBS approaches, thus facilitating improvement in an overall image segmentation result. Further, direct integration into a MBS framework may be facilitated by the proposed concept(s), thus enabling the omission of additional pre-processing steps that may otherwise be required by conventional/alternative model initialisation approaches.

Proposed embodiments may, for example, provide a medical image segmentation algorithm that delivers a robust surface-based segmentation result for strongly varying target organ variations. Accordingly, embodiments may be used in relation to medical images so as optimize implementation or allocation of medical assessment, therapy and/or treatment for a subject. Such embodiments may support clinical planning. Improved Clinical Decision Support (CDS) may therefore be provided by proposed concepts.

In some embodiments, placing the model within the detected set of landmarks may comprise interpolating the model shape based on the determined connections for the landmarks. Thus, based on required distances from the landmarks to model for example, the model shape can be placed into the landmark set. For instance, during placement, the model may be deformed (while considering the model's internal energy to only allow realistic deformations for example) in such a way that the connector length (i.e. the relative position from model to landmarks) is maintained.

Accordingly, in some embodiments, placing the model within the detected set of landmarks may comprise deforming the model while, for each landmark, maintaining the position of the landmark relative to the model represented by its respective connection. Furthermore, such a process of deforming the model may be based on the internal energy of the model.

In some embodiments, defining a set of landmarks may comprise positioning the set of landmarks based on a predefined structure. For instance, given a segmentation mesh, the set of landmarks may be placed into the model space either outside, inside, or on the mesh surface). Put another way, positioning the set of landmarks may comprise distributing the set of landmarks within, in or around a boundary of the predefined structure. By evenly or uniformly distributing the landmarks around the target structure outline (with arbitrary distance) for example, it may be ensured that the initialisation will be within the capture range of the model, thereby ensuring a successful segmentation.

In some embodiments, a landmark's respective connection may comprise a linear connection between the landmark and the model. By way of example, the linear connection may define the shortest distance between the landmark and the model. In other examples, the linear connection may not define the shortest distance between the landmark and the model, but may instead define a predetermined distance and/or position relative to the model.

According to another aspect of the invention, there is provided computer-implemented method of model-based image segmentation, comprising initialising a model for model-based segmentation of the medical image according to a proposed embodiment.

According to another aspect of the invention, there is provided a computer program comprising code means for implementing a method according to an embodiment when said program is run on a processing system.

According to another aspect of the invention, there is provided a system for initialising a model of a model space for model-based segmentation of an image. The system comprises: a definition component configured to define a set of landmarks in a model space; an analysis component configured to determine, for each of the landmarks, a respective connection to the model of the model space, the connection being representative of the position of the landmark relative to the model; a detection component configured to detect the set of landmarks in the image; and a placement component configured to place the model within the detected set of landmarks based on the determined connections for the landmarks.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 is a flow diagram of a computer-implemented method for initialising a model of a model space for model-based segmentation of an image according to an embodiment of the invention;
FIGS. 2A and 2B illustrate an example of a proposed embodiment of initialising a model for model-based segmentation of an image; and
FIG. 3 is a simplified block diagram of a system for initialising a model of a model space for model-based segmentation of an image according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Proposed are concepts for model initialisation for model-based segmentation (MBS) of an image. Such concepts may employ a set landmarks to initialise a segmentation mesh. As a result, embodiments need not be limited to predefined model transformations, but can initialise a segmentation mesh with arbitrary shape. Embodiments may therefore facilitate an image segmentation algorithm that not only delivers a robust surface-based segmentation result but also does so for a segmentation mesh with arbitrary shape.

In particular, proposed concepts may an MBS algorithm for medical image segmentation that delivers a robust result for strongly varying target organ variations. Accordingly, embodiments may be used in relation to medical images and/or provide improved Clinical Decision Support (CDS).

According to a proposed concept, there is provided a landmark-based model initialization approach for model-based organ segmentation. Such an approach uses a set of landmarks (e.g. distributed over the image) to initialize the MBS model, wherein the spatial positions of the landmarks can be correlated with respect to the MBS model. After detecting the landmarks in the image to be segmented, embodiments may place the model shape into the landmark set in such a way that relative positions from model to landmarks are maintained. Once the model is placed into the image, the result can be used as initialization to consequently trigger a MBS pipeline.

Illustrative embodiments may, for example, be employed in model-based image segmentation systems, such as in medical imaging analysis systems.

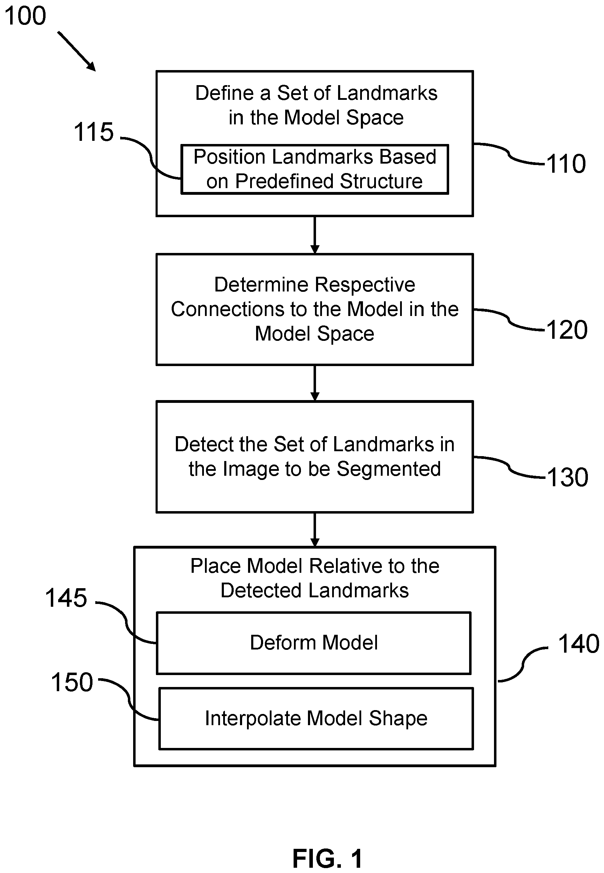

Referring to Figure 1, there is depicted a flow diagram of a computer-implemented method 100 for initialising a model of a model space for model-based segmentation of an image according to an embodiment of the invention.

Here, the image may, for example, be a volumetric medical image. For example, the volumetric image may be a computed tomography (CT) image, a magnetic resonance (MR) image, a nuclear medicine image, such as a positron emission tomography (PET) image or a single photon emission computed tomography (SPECT) image, or a volumetric ultrasound image.

The method begins with step 110 of defining a set of landmarks in a model space. In this example, the step 100 defining the set of landmarks comprises the step 115 of positioning the set of landmarks based on a predefined structure (e.g. a target/reference organ structure). Here then set of landmarks are positioned by distributing the set of landmarks uniformly within, in or around a boundary of the predefined structure. In this way, the landmarks are placed into the model space.

The method then comprises the step 120 of, for each of the landmarks, determining a respective connection to the model in the model space. Here, a landmark's respective connection comprises a linear connection between the landmark and the model, and is thus representative of the position of the landmark relative to the model. In this example, a linear connection defines the shortest distance between the landmark and the model. It will therefore be a linear connection defined by a line connecting the landmark to the closest point of the model surface, the line being perpendicular to the tangent of the closest point of the model surface. However, in other examples, the connection need not be the shorted distance.

Then, in step 130, the landmarks are detected in the image to be segmented. For this, a landmark detector (using any known algorithm) identifies the set of landmarks in the image to segment. Several approaches for landmark detection/localization in medical images are described in existing literature. Detailed description of landmark detection is therefore omitted from this description for conciseness.

After detecting the landmarks, the method continues to the step 140 of placing the model within the detected set of landmarks (so as to 'activate' or 'initialise' the model). Such placement of the model is based on the determined connections for the landmarks. In particular, placing 140 the model relative to the detected set of landmarks comprises the process 145 of deforming the model while maintaining the position of the landmarks relative to the model represented by their respective connections. This may, for example, be done while considering the model's internal energy to only allow realistic deformations. Further, placing 140 the model within the detected set of landmarks may comprise the step 150 of interpolating the model shape based on the determined connections for the landmarks (e.g. so as to keep the connection lengths stable).

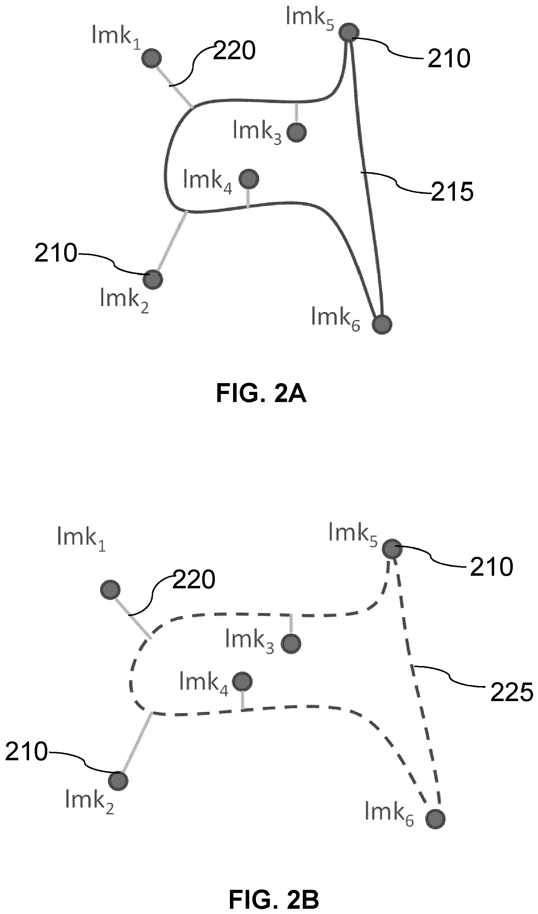

By way of further description, an example of a proposed embodiment of initialising a model for model-based segmentation of an image will now be described with reference to Figures 2A and 2B.

Figure 2A depicts a model space, wherein a plurality of landmarks 210 are connected to the model 215 via linear connectors 200.

Figure 2B depicts the model space wherein the model has been initialised for the image according to the embodiment, wherein the segmentation mesh 225 is interpolated into the landmark set by keeping the connector lengths stable.

First, a set of landmarks is defined and then their spatial position with respect to the model 215 is correlated. Figure 2A illustrates this for a schematic vertebra model. Given a segmentation mesh 225, the set of detectable landmarks 210 is placed into the model space, either outside (e.g. lmk₁ and lmk₂), inside (e.g. lmk₃ and lmk₄), or on (e.g. lmk₅ and lmk₆) the mesh surface 225.

The spatial correlation, i.e. the relative position of the landmarks 210 with respect to the model 225 may be represented by connecting the landmarks 210 with the model using linear connectors 200.

Referring now to Figure 2B, during application, a landmark detector (using any known landmark detection algorithm) firstly detects the landmarks 210 in the image to be segmented. Several approaches for landmark localization in medical images are known and described in the literature. Description of such landmark detection algorithms is therefore omitted from this description.

Once the landmarks 210 are detected, the model can be "activated", i.e. based on the required distances from landmarks 210 to model, the model shape is placed into the landmark set. During placement, the model is deformed (e.g. while considering the model's internal energy to only allow realistic deformations) in such a way that the connector 220 lengths (i.e. the relative positions from model to landmarks) is maintained.

Once the model is placed into the image so to as obtain a modified model 225, this model 225 is use as an initialization to consequently trigger a MBS pipeline.

Here, it is noted that, if the detected landmarks are evenly distributed around the target outline (e.g. with arbitrary distance), the initialization should be within a capture range of the model, and a successful segmentation can thus be ensured.

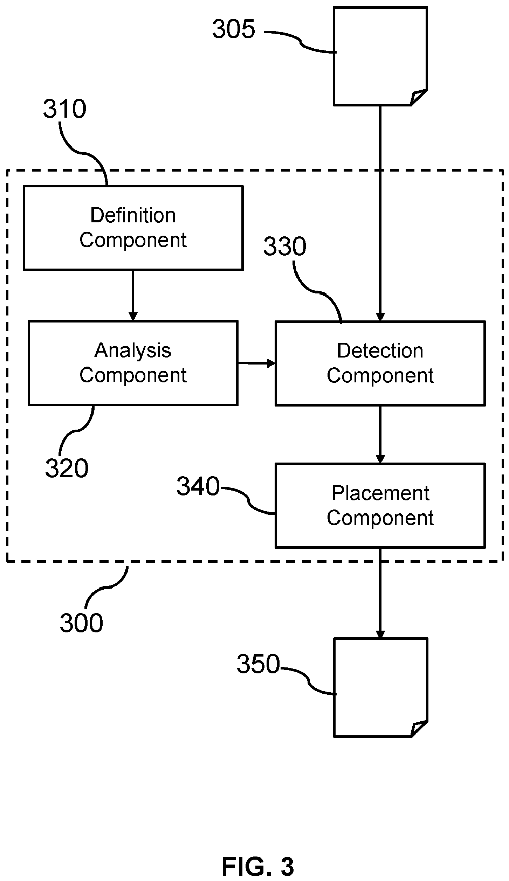

Figure 3 illustrates a system 300 for initialising a model of a model space for model-based segmentation of an image 305 according to exemplary embodiment.

The system 300 comprises a definition component 310 that is configured to define a set of landmarks in a model space. Here, the definition component 310 is configured to position the set of landmarks based on a predefined structure, such as a target/reference structure for example.

An analysis component 320 of the system is then configured to determine, for each of the landmarks, a respective connection to the model of the model space. A connection is representative of the position of the landmark relative to the model. For instance, in this example, a landmark's respective connection comprises a linear connection between the landmark and the model.

The system 300 further comprises a detection component 330 that is configured to detect the set of landmarks in an image 305 to be segmented (e.g. received via in input interface of the system).

Based on the determined connections for the landmarks from the detection component 330, a placement component 340 of the system 300 is configured to place the model within the detected set of landmarks. Specifically, the placement component 340 of this example interpolate the model shape based on the determined connections for the landmarks. The placement component 340 is also configured to output the placed (i.e. initialised) model 350 via an output interface of the system 300. Thus, by way of example, the output model 350 may be provide to a MBS pipeline.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented of initialising a model of a model space for model-based segmentation of an image, the method comprising:
defining a set of landmarks in a model space;
for each of the landmarks, determining a respective connection to the model in the model space, the connection being representative of the position of the landmark relative to the model;
detecting the set of landmarks in the image; and
placing the model within the detected set of landmarks based on the determined connections for the landmarks.

2. The method of claim 1, wherein placing the model within the detected set of landmarks comprises:
interpolating the model shape based on the determined connections for the landmarks.

3. The method of claim 1 or 2, wherein placing the model within the detected set of landmarks comprises:
deforming the model while, for each landmark, maintaining the position of the landmark relative to the model represented by its respective connection.

4. The method of claim 3, wherein deforming the model is based on the internal energy of the model.

5. The method of any of claims 1 to 4, wherein defining a set of landmarks comprises:
positioning the set of landmarks based on a predefined structure.

6. The method of claim 5, wherein positioning the set of landmarks comprises distributing the set of landmarks within, in or around a boundary of the predefined structure.

7. The method of any of claims 1 to 6, wherein a landmark's respective connection comprises a linear connection between the landmark and the model.

8. The method of claim 7, wherein the linear connection defines the distance between the landmark and the model.

9. A computer-implemented method of model-based segmentation of a medical image comprising: initialising a model for model-based segmentation of the medical image according to any of claims 1 to 8.

10. A computer program comprising computer program code means adapted, when said computer program is run on a computer, to implement the method of any of claims 1 to 9.

11. A system for initialising a model of a model space for model-based segmentation of an image, the system comprising:
a definition component configured to define a set of landmarks in a model space;
an analysis component configured to determine, for each of the landmarks, a respective connection to the model of the model space, the connection being representative of the position of the landmark relative to the model;
a detection component configured to detect the set of landmarks in the image; and
a placement component configured to place the model within the detected set of landmarks based on the determined connections for the landmarks.

12. The system of claim 11, wherein the placement component is configured to interpolate the model shape based on the determined connections for the landmarks.

13. The system of claim 11 or 12, wherein the placement component is configured to deform the model while, for each landmark, maintaining the position of the landmark relative to the model represented by its respective connection.

14. The system of any of claims 11 to 13, wherein the definition component is configured to position the set of landmarks based on a predefined structure.

15. The system of any of claims 11 to 14, wherein a landmark's respective connection comprises a linear connection between the landmark and the model.
